# EUROPEAN PATENT APPLICATION

(11) **EP 1 493 415 A2**
(43) Date of publication of application: **05.01.2005**
(21) Application number: 04253888.4
(22) Date of filing: 29.06.2004
(51) Int. Cl.: A61F 13/511

(54) **Embossed absorbent article**

(30) Priority: 30.06.2003 US 609739
(71) Applicant: McNeil-PPC, Inc., Skillman, New Jersey 08558 (US)
(72) Inventor: Hull, Raymond J. Jr, Hampton New Jersey 08827 (US); Schmidt, Brian D., Little Falls New Jersey 07424 (US)
(74) Representative: Mercer, Christopher Paul

(57) **Abstract**

An absorbent article including: a) a body facing surface and b) a garment facing surface wherein at least one embossment is provided on a surface of a material of the absorbent article and the embossment comprises a raised portion having an upper surface that is formed within a depressed area between at least two sidewalls, wherein the surface of the material opposite the embossing pattern is substantially flat.

## Description

### Field of the Invention

The invention relates to embossing of an absorbent article. In particular, it is directed to embossing devices, methods of embossing, embossing patterns, and the like wherein the embossing pattern provides a depressed area having a textured portion therein.

### Background of the Invention

Disposable absorbent articles, such as, pantiliners, sanitary napkins, interlabial devices, adult incontinence devices, bandages, wipes, diapers and the like are well known. These articles typically have a fluid permeable body-facing side and fluid impermeable garment facing side. Additionally, such articles may include an absorbent layer for retaining fluids therebetween.

Absorbent articles having patterns imposed upon their surfaces are now on the market. For example, sanitary protection articles, such as, napkins, pantiliners, and incontinence devices, typically have a cover of nonwoven material having a pattern of depressed areas embossed into the surface in such configurations as flowers or other feminine designs. Other patterns may take the form of various geometric shapes, such as, circles, diamonds, squares, curves, or other stylized figures, such as, stars, spots, or the like.

While articles are embossed mainly for decorative purposes, embossing may communicate or provide a function to the user of such an article. For example, it is known that embossing functions in impeding or directing fluid flow. See for example - four-wall patent.

Embossing methods for absorbent articles are well known. For example, U.S. Pat. No. 4,518,451 discloses an embossed pantiliner where the body facing side of the absorbent body has imposed therein a pattern of relatively deep depressed areas whereas the longitudinal edges have imposed therein a pattern of relatively shallow depressed areas. This results in a pantiliner that has aesthetically effective embossing while providing comfortable longitudinal edges. U.S. Pat. No. 4,623,340 discloses an absorbent pantiliner provided with a pattern of depressed areas. The outer cover of the pantiliner is made from a relatively light opaque sheet material and the interior layer is at least partially thermoplastic and relatively dark colored. When the cover is embossed, the depressed areas appear darker than the unembossed areas.

Embossing is also done to provide a visual cue to show differences in the material surface.

To be aesthetically effective, embossing has up until the present invention, been relatively deep; e.g., the depressed areas must be permanently depressed to a degree, which represents a major portion of the thickness of the product. Shallow embossing has proven to be visually uneffective. Unfortunately, the effect of extreme compression for deep embossing is to produce a rather dense, harsh, and inflexible material that may be a source of discomfort to the user due to chafing, especially if contacting the thighs.

What is needed therefore is an embossment that is aesthetically effective and avoids the discomfort associated with extreme compression. This invention provides such an embossment,

### Summary of the Invention

The present invention is directed to an absorbent article comprising, consisting of and/or consisting essentially of:
a) a body facing surface and
b) a garment facing surface
wherein at least one embossment is provided on a surface of a material of the absorbent article and the embossment comprises a raised portion having an upper surface that is formed within a depressed area between at least two sidewalls, wherein the surface of the material opposite the embossing pattern is substantially flat.

The present invention is also directed to an embossment in a material comprising, consisting of and/or consisting essentially of at least one depressed area and a raised portion having an upper surface that is formed within the depressed area between at least two sidewalls, wherein the surface of the material opposite the embossing pattern is substantially flat.

The present invention is further directed to a device for embossing comprising, consisting of and/or consisting essentially of a substantially flat female roll and a male roll configured to provide at least one embossment comprising at least one depressed area and a raised portion having an upper surface that is formed within the depressed area between at least two sidewalls, wherein the surface of the material opposite the embossing pattern is substantially flat.

### Brief Description of the Drawings

FIG. 1 shows a perspective view of an embodiment of the present invention;
FIG. 2 shows a plan view of the embodiment shown in Fig. 1;
FIG. 3 shows a cross-sectional view taken along line BB of FIG. 2;
FIG. 4 shows a perspective view of an embodiment of the present invention;
FIG. 5 shows a plan view of the embodiment shown in FIG. 4;
FIG. 6 shows a cross-sectional view taken along line AA of FIG. 5;
FIG. 7 shows a perspective view of an embodiment of the present invention;
FIG. 8 shows a plan view of the embodiment shown in FIG. 7;
FIG. 9 shows a cross-sectional view taken along line CC of FIG. 8;
FIG. 10 shows a perspective view of an embodiment the present invention;
FIG. 11 shows a plan view of the embodiment shown in FIG. 10;
FIG. 12 shows a cross-sectional view taken along line DD of FIG. 11;
FIG. 13 shows a perspective view of an embodiment of the present invention;
FIG. 14 shows a plan view of the embodiment shown in FIG. 13;
FIG. 15 shows a cross-sectional view taken along line EE of FIG. 14;
FIG. 16 shows a perspective view of an embodiment of the present invention;
FIG. 17 shows a plan view of the embodiment shown in FIG. 16;
FIG. 18 shows a cross-sectional view taken along line FF of FIG. 17;
FIG. 19 shows an enlarged view of area X of FIG. 18; and
FIG. 20 shows a plan view of an embodiment of the present invention.

### Detailed Description of the Invention

As used herein, the term "embossed pattern" shall mean the portion of the material that is deformed by an embossing device.

As used herein, the term "texture" shall mean a three-dimensional surface.

As used herein, the term "textured portion" shall mean that portion of the embossed pattern that has texture imparted thereon by the embossing device.

The present invention discloses an embossed product and a method to enhance the aesthetic effectiveness of embossments, particularly when incorporated in absorbent articles. Such an embossed absorbent article of the present invention has depressions that have been found to enhance the aesthetic effectiveness of the embossing by incorporating at least one textured portion in the depression. Additionally, the depressed areas can have further improved aesthetic effectiveness by coating a portion or all of the depression, ridge or both with a coating material. In addition, the embossments of the present invention can control and direct fluid flow about an absorbent article.

In general, the embossing methods employed by this invention may be done by any methods known in the art using an embossing tool of the present invention.

Embossing is typically done by feeding or placing material between the embossing tool and a back-up plate or roller, wherein the embossing tool imparts deformation onto selected portions of the material due to the shape of the embossing tool. The deformation of the material results from not only the shape of the embossing tool, but the stress put on the material by the embossing tool.

In this invention, the area of the embossing tool that imparts the embossed pattern onto the material includes more than one elevation. It is the use of multiple elevations that provide texture within the embossed pattern of the material.

The back-up plate or roll may be smooth such when used in conjunction with the embossing tool, only one surface of the material has an embossed pattern.

The embossing tool and the back-up plate or roll according to this invention may be made of metal, polymers, rubber, and any material having greater rigidity as compared to the material being deformed.

Embossing can be done with or without heating. For example, if the material is lofty and requires heat to set the embossing pattern such that the material does not regain its original surface smoothness, heat may be employed to bond the embossed pattern.

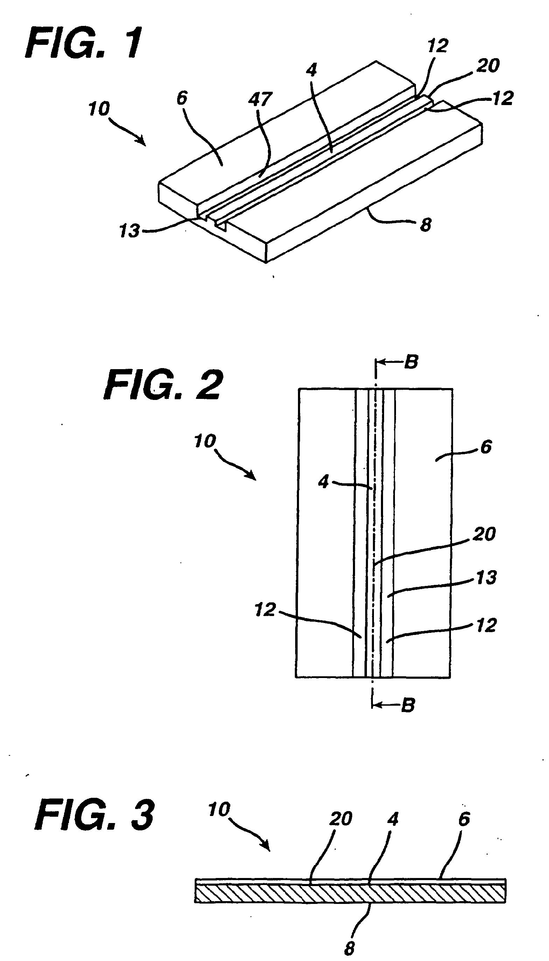

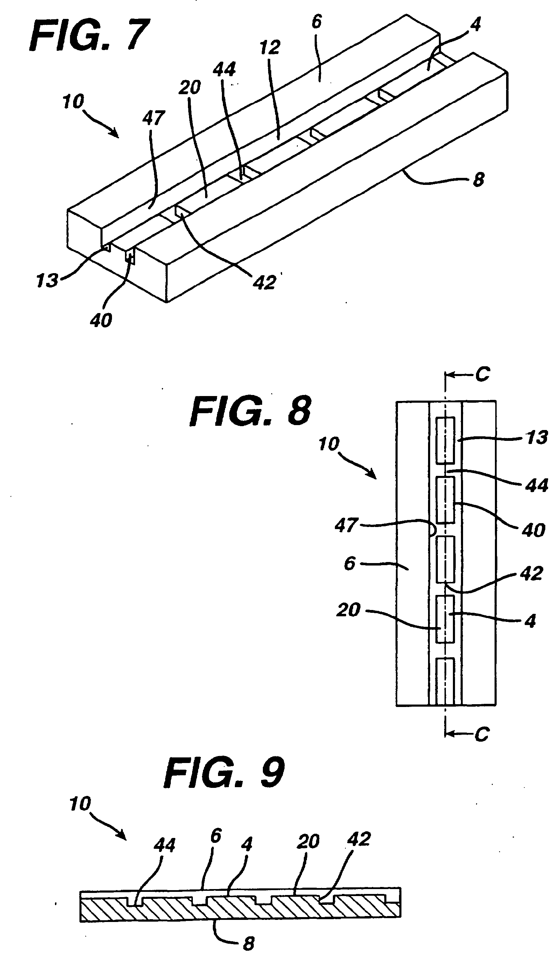

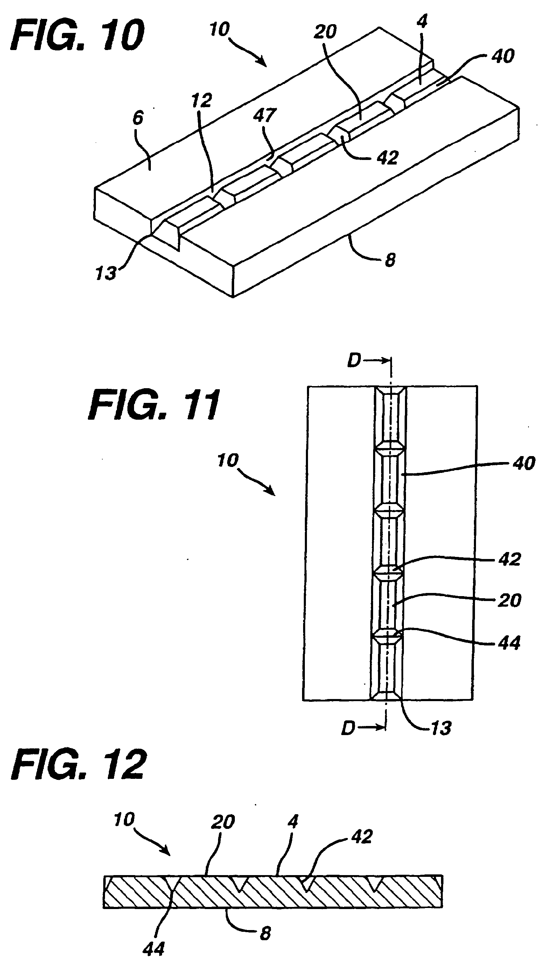

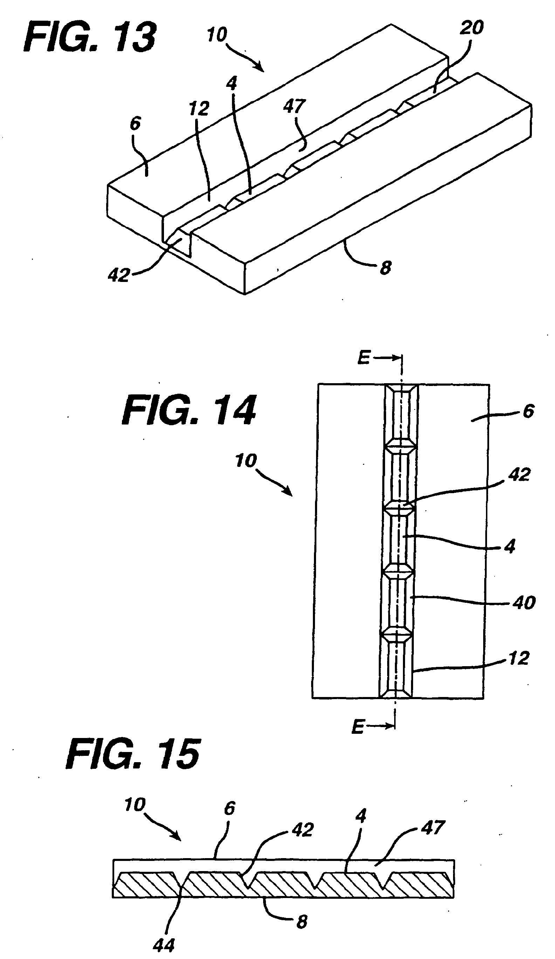

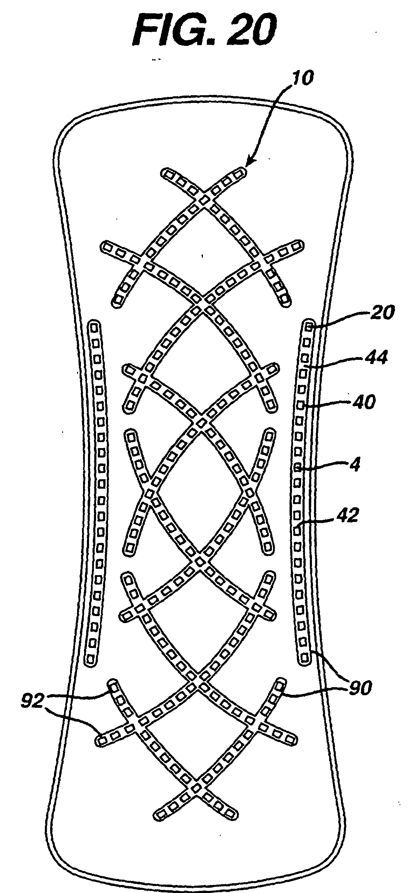

Turning to the Figures, Figures 1-19 depict various textures of the embossed patterns. Figure 20 depicts an article having an embossed pattern thereon.

As seen in Figures 1, 4, 7, 10, 13, and 16, a material having an embossed upper such that embossed pattern 10 has texture including depressed areas 12 and raised portions 20. Note that the lower surface 8 is unaffected by the embossing of upper surface 6, in other words the lower surface 8 is not distorted.

Each embodiment depicted in the Figures 1-15 provides textured embossing that includes at least two depressed areas 12 and raised portion 20.

Each depression 12 includes a longitudinal depressed area floor 13, an optional lateral depressed area floor 44, and depressed area sidewalls 47 extending from longitudinal depressed area floor 13 to upper surface 6.

Each raised portion 20 includes raised portion upper surface 4, raised portion longitudinal side walls 40 extending from raised portion upper surface 20 to longitudinal depressed area floor 13, and raised portion lateral sidewall 42 extending from raised portion upper surface 20 to lateral depressed area floor 44.

While the embossed pattern shown in these figures appear to depict the depressions 12 each having the same depth, it is not necessary. For example, one depression 12 may be more shallow than another depression 12 on the embossed pattern 10. Additionally, a single raised portion 20 may include different heights for raised portion upper surface 4. Where a plurality of raised portions 20 are present, the height of the raised portions 20 defined by the distance from raised portion upper surface 4 to longitudinal depressed area floor 44, lateral depressed area floor 12, or both may be the same or different for each raised portion 20. In addition, in such embodiment, the height of a single raised portion 20 as defined above may be different within the same raised portion 20.

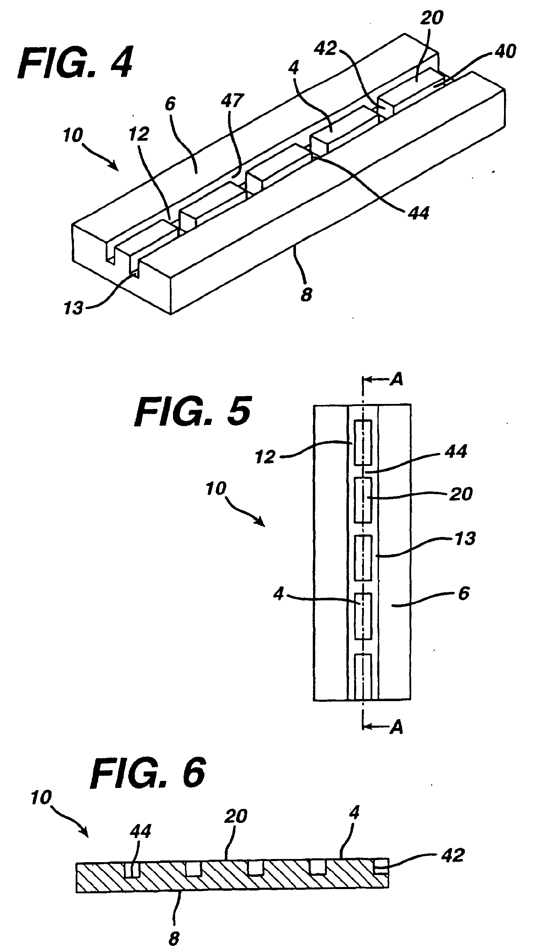

As shown in Figures 4, 7, 10 and 13, raised portions 20 include raised portion upper surface 4, raised portion longitudinal side walls 40 extending from raised portion upper surface 20 to longitudinal depressed area floor 13, and raised portion lateral sidewall 42 extending from raised portion upper surface 20 to lateral depressed area floor 44. In one embodiment, raised portion longitudinal side walls 40 and raised portion lateral sidewall 42 are substantially perpendicular to lower surface 8. In other embodiments, raised portion longitudinal side walls 40 may be parallel to each other or in a non-uniform alignment to each other. Similarly, raised portion lateral sidewall 42 may be parallel to each other or in a non-uniform alignment to each. An example of parallel raised portion longitudinal side walls 40 can be seen in Figure 20.
Additionally, raised portion longitudinal side walls 40 do not need to be perpendicular to raised portion lateral sidewall 42.

Raised portion 20 may be any shape, e.g, cone, cube, rectangle, rounded, e.g., oval, and the like. Similarly, raised portion upper surface 4 may be any shape, e.g., flat, curved, pointed, irregular planes and combinations of such shapes. Where raised portion 20 is a cone shape, raised portion upper surface 4 contains an apex.

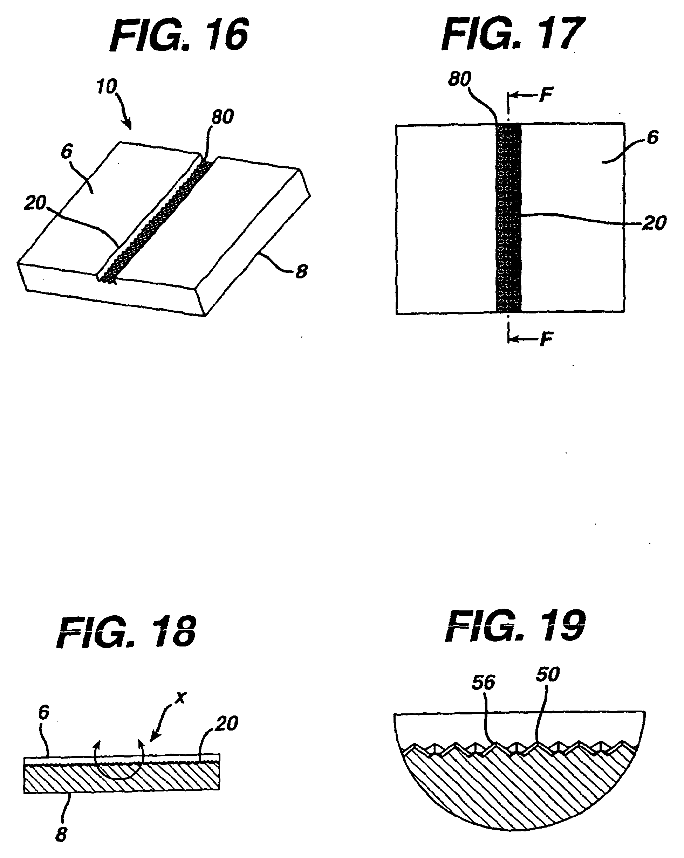

Another embodiment of the invention can be seen in Figures 16-19. In this embodiment, depressed area 80 is made of a series of raised portions 20 shown as elevations 50. Valleys 52 (not shown) are between elevations 50. The elevations 50 and valleys 52 are connected by walls 56. As depicted in Figure 19, walls 56 do not need to be perpendicular to lower surface 8, but they may be.

The embossed pattern can be a continuous pattern, such as, a line; or a discrete (interrupted) pattern, such as, geometric shapes, e.g., rectangles, diamonds, squares, circles; letters; numbers; images; or combinations thereof.

In one embodiment of the invention, the pattern can be embossed onto the body facing surface of a disposable absorbent article. Such articles include, pantiliners, sanitary napkins, interlabial devices, adult incontinence devices, bandages, wipes, and diapers.

In the embodiment shown in Figure 20, a series of raised portions 20 within depressed areas combine to form a single pattern 90. Multiples of this pattern 92 may be combined on the body facing surface of the absorbent article as seen in Figure 20. Such multiple patterns 92 may include, for example, a series of hatch marks, criss-crosses, bars, knurled, waves, spirals, swirls, letters, numbers, logos, pictures, and the like. Other patterns are possible and are included in this invention.

When used in an absorbent article, embossed pattern 10 should have sufficient depth to be visually effective. The width of embossed pattern 10should also be sufficient to be visually effective. In particular, in sanitary protection articles, it has been found that the width of embossed pattern 10 should be at least from about 1 mm to about 25 mm, from about 5 mm to about 10 mm. All numbers in the above ranges are expressly disclosed.

The depth of embossed pattern 10 is dependent on the material that is embossed. For example, in some material, deep embossing may enhance the visibility of the pattern but results in stiffer material. This is an undesired outcome for a sanitary protection article due to inherent discomfort caused by a stiffer material. When a pantiliner is embossed according to the present invention, the embossing pattern 10 depth should be from about 0.1 to about 3 mm. When a sanitary napkin is embossed according to the present invention, the embossing pattern 10 depth should be from about 0.1 to about 15 mm. This can be seen in Figures 3, 6, 9, 12, and 15. Raised portion 20 may be any height, as defined above, that is shorter than depressed area side wall 47. All numbers in the above ranges are expressly disclosed.

The embossed pattern 10 is formed, for example, by embossing a substrate, e.g., a web, with a system of embossing rolls. One of the rolls may be a metal roll, e.g., steel, aluminum, and the like, whose surface is engraved with a pattern of protrusions, which is commonly referred to as a male embossing roll. The land area of the protrusions is textured, that is, the land area has a pattern that is contained on its surface. The male embossing roll mates with the female embossing roll forming a series of depressions into the surface plane of the substrate, e.g, a web. The rolls are aligned with a gap, i.e., fixed or floating, between them such that a portion of the material is pattern free. As a result of the textured land area of the male embossing roll, the depression will have a distinctive pattern.

Alternately, the embossing rolls may be reversed. The female roll may be steel and may include depressions having texturized portions.

While rolls are exemplified above, any method of embossing, e.g., plates, can be used as well.

Upper surface 6, raised portion upper surface 4, or both may be printed or otherwise coated. Suitable coating materials used to produce include, but are not limited to, hot melt coatings; natural rubber; synthetic rubber; polyolefins, such as, polyethylene and polypropylene; ethylene vinyl acetate; and thermoplastic elastomers. Colorants, pigments, or fragrances may be combined with the coating materials.

Suitable hot melt coatings for include HL-7471 W, from H.B. Fuller Co., St. Paul, MN, and REXTAC amorphous polyolefins, available through Huntsman Chemical. Hot melt coatings containing from about 15% to about 100% olefin polymer or a block copolymer, from about 0% to about 60% tackifying resin, and from about 0% to about 50% wax may be useful. In addition, the use of fillers in the coating may also be beneficial to reduce costs, add process benefits, e.g., thixotropy. or to provide masking or whitening benefits. Examples of such fillers include, but are not limited to, fumed silica, such as, Cabosil, from Cabot Corp., calcium carbonate, titanium dioxide, zinc oxide, magnesium oxide, wood flour, or diatomaceous earth.

Additionally, water dispersible sulfopolyester hot melt adhesive (available from National Starch, Bridgewater, NJ ) can be used. Eastman AQ water-dispersible polyesters (from Eastman Chemical) may also be used.

Suitable olefin polymers include polymers: a) wherein the olefin polymer is a homopolymer of ethylene, propylene, n-butene, butylene or isobutylene, with a melt flow index from 0.5 to 2500, such as Ateva® polymers from AT plastics; Escorene® and Vistanex® polymers from Exxon Chemical, Duraflex® polymers from Shell Chemical, Epolene® polymers from Eastman Chemical, and Vestoplast® polymers from Creanova; b) wherein the olefin polymer is a copolymer of ethylene and a comonomer, such as vinyl acetate, acrylic acid, methacrylic acid, ethyl acrylate, methyl acrylate, n-butyl acrylate vinyl silane or maleic anhydride, such as Ateva™ polymers from AT plastics, Elvax® polymers from DuPont, Escorene® and Optema® polymers from Exxon Chemical, and Primacor® polymers from Dow Chemical; and c) wherein the olefin polymer is a terpolymer of ethylene and co-monomers, such as vinyl acetate, acrylic acid, methacrylic acid, ethyl acrylate, methyl acrylate, n-butyl acrylate vinyl silane or maleic anhydride, such as Ateva® polymers from AT plastics, Nucrel® polymers from DuPont, and Escor® polymers from Exxon Chemical.

Suitable block copolymers include block copolymers having a linear or a radial structure such that the structure (A--B), where A consists essentially of a polyvinylarene block, and B consists essentially of poly(monoalkenyl) block, and x denotes the number of polymeric arms, where x is greater than or equal to one are also useful. Block B may be selected from conjugated diene elastomers such as polybutadiene or polyisoprene and hydrogenated elastomers such as ethylene-butylene or ethylene- propylene. Suitable examples of these types of polymers include Kraton® elastomers from Shell Chemical Company, Vector® elastomers from Dexco, Solprene® elastomers from Enichem Elastomers and Stereon® from elastomers Firestone Tire & Rubber Co. When the hot melt coatings contain block copolymers, it is preferable for the coating to contain from about 15% to about 50% block copolymer.

Suitable tackifying resins include any compatible resin or mixture thereof selected from the group consisting of a) natural and modified rosins; b) glycerol and pentaerythritol esters of natural and modified rosins; c) polyterpene resins; d) copolymers and terpolymers of natural terpenes; e) phenolic modified terpene resins and the hydrogenated derivatives thereof; f) aliphatic petroleum resins and the hydrogenated derivatives thereof; g) aromatic petroleum resin and the hydrogenated derivatives thereof; and h) aliphatic/aromatic petroleum resins and the hydrogenated derivatives thereof, such as Foral® resin, Staybelite® resin, Poly-pale® resin, Permalyn® resin, Pentalyn® resin, Adtac® resin, Piccopale® resin, Piccotac® resin, Hercotac® resin, Regalrez® resin, and Piccolyte® resin from Hercules, Escorez® resin from Exxon Chemical, Wingtack® resin from Goodyear Tire & Rubber Co., Arkon® resin from Arakawa Chemicals, Zonatac® resin, Zonarez® resin and Zonester® resin from Arizona Chemical and Nevtac® resin from Neville Chemical Company.

Suitable waxes include, but are not limited to, paraffins, Fischer-Tropsch, microcrystalline waxes, and combinations thereof. Suitable microcrystalline waxes include, but are not limited to, BE SQUARE 175 microwax, available from Bareco Division, Petrolite Corporation, and M-5165 from Moore & Munger, Shelton, CT. Suitable polyethylene waxes include, but are not limited to, H-101 from Exxon Chemical, Houston, TX. Suitable Fischer-Tropsch waxes include, but are not limited to, Paraflint Wax from Schumann Sasol, Hamburg, Germany.

The coatings can be deposited on the surface of a substrate by any method known in the art, including, but not limited to, gravure printing, screen printing or coating, kiss coating, porous roll printing, flexo printing, reverse roll coating, transfer coating and the like.

The coating can be deposited on the floor area, at least one of the side walls of the embossed area, or both. Such methods are disclosed in co-pending application entitled ENHANCED EMBOSSING AND RELATED METHODS, USSN 10/ , filed concurrently herewith, which is commonly owned and incorporated herein by reference.

Absorbent articles typically include a cover or a body facing liquid pervious surface and a backsheet or a garment facing liquid impervious layer. Additionally, an absorbent core, transfer layer, construction and positioning adhesive, and release paper may be included in the absorbent article construction.

### Cover

The cover of an absorbent article includes the body-facing surface. In one embodiment, the cover may be a layer of material that is juxtaposed on the absorbent core. In separate embodiment, the cover may be a coating deposited on the absorbent core. Such a coating may be a continuous surface or may include a plurality of discrete entities. Materials used for coating are described above as being useful for coating upper surface 6 and raised portion upper surface 4.

As known by those skilled in the art, the cover may be formed from any fluid pervious material that is comfortable against the skin and permits fluid to penetrate to the absorbent core, which retains the fluid. The cover should retain little or no fluid to provide a relatively dry surface next to the skin when in use. The cover can be a single sheet or layer of material having a width sufficient to form the body-facing surface of the absorbent article.

A variety of cover materials are known in the art, and any of these may be used. For instance, the cover has been made from fibrous non-woven fabrics made of fibers or filaments of polymers, such as polyethylene, polypropylene, polyester, or cellulose, and combinations or mixtures thereof. The fiber or filament can be single denier or multidenier.

Other materials used in making covers include gauze or for example, a nonwoven material such as the ones described in U.S. Pat. No. 3,554,788 (Fechillas), any known porous material with a suitable body contacting surface, including, but not limited to nonwoven webs, apertured films, plastic nets, and the like. Previously, the cover could also be made from a fibrous nonwoven composite of bicomponent fibers and pulp fluff.

Alternatively, the cover may be formed from an apertured polymeric film. In addition, such a film may be treated with a surfactant to increase hydrophillicity.

Additionally, the material used to form the cover should be hydrophilic, which can be obtained by any methods known to those skilled in the art, such as surface treatment, surface spraying, or incorporation of a surfactant into the material.

### Transfer Layer

The absorbent article of the present invention may have an optional transfer layer. The transfer layer may be made of any known material that will take up fluid and then distribute and release it to an adjacent absorbent core or layer for storage. Transfer layers often have a relatively open structure that allows for movement of fluid within the layer. Suitable materials for such transfer layers include fibrous webs, resilient foams, and the like.

The transfer layer is able to accept fluid and allow passage of the fluid through its mass to be absorbed by an adjacent absorbent core. Thus, transfer layers that are made of hydrophobic, nonabsorbent fibers may be able to accept large volumes of fluid into interfiber void spaces while the fibers themselves do not absorb any significant quantities of fluid. Likewise, open-celled foam structures that are made from nonabsorbent materials may also absorb fluid into the cells of the foam. The walls of the cells, however, do not absorb any fluid. The cumulative spaces within the transfer layer, i.e., the interfiber void spaces in the fibrous transfer layer or the open cells in the foam transfer layer, function much like a container to hold fluid.

Transfer layers that are made from webs of mostly absorbent fibers absorb the fluid as it enters the structure and do not distribute it throughout the rest of the structure as efficiently as webs containing non-absorbent materials. Preferred transfer layer fibrous webs include nonabsorbent materials to provide void volume and to allow for free movement of fluid through the structure. Examples of preferred materials include polypropylene, polyethylene, polyester, bicomponent materials, nylon and mixtures or combinations thereof.

The transfer layer does not have to be apertured film; it can be any other nonwoven material, such as, foam or netting, which transports fluids.

The cover layer can be joined or laminated to the transfer layer by any methods known in the art such as fusion bonding, adhesive attachment, or by any other methods for securing layers together. Fusion bonding includes heat bonding, ultrasonic bonding, and the like.

In a useful embodiment, the cover and transfer layers are joined together by first applying adhesive to the underside of the cover material and placing the cover web onto the transfer layer material. While any adhesive may be used, such as any non-pressure sensitive adhesive, a preferred adhesive is D 1280 BE (available from Fuller Co., Germany).

In one embodiment, the composite layer formed from the cover and transfer layers may be further processed.

### Absorbent Core

The absorbent core or layer of the present invention may contain any known absorbent materials including, but not limited to, absorbent fibers, such as cellulose fibers, including, but not limited to wood pulp, regenerated cellulose fibers, and cotton fibers, rayon fibers and the like; superabsorbent fibers or particles; other naturally occurring absorbent materials, such as peat moss; and other synthetic absorbent materials, such as foams and the like. The absorbent layer may also include one or more of the following: thermoplastic binder fibers, latex binder, perfumes, oils or odor-controlling compounds. Additionally, the absorbent layer may be a mixture of two or more types of thermoplastic fibers having different melting points. Bicomponent fibers, fibers with an inner core of a thermoplastic fiber, e.g., polyester, surrounded by an outer sheath of thermoplastic, e.g., polyethylene, having a melting point much lower than the core, have been found to be the best fibers to work with from processing and performance standpoints. Upon application of heat and pressure sufficient to melt at least one of the fiber types, the remaining unmelted fibers will be thermobonded or fused together into a porous web.

Cellulosic pulp fibers can also be included with thermoplastic fibers. Since thermoplastic fibers, without further treatment, are essentially hydrophobic, the absorbent layer will not effectively draw fluid away from the composite cover and transfer layer absent some hydrophilic material. It is important to have sufficient pulp to absorb fluid.

In a particularly useful embodiment, the absorbent layer is made from airlaid pulp and includes about 15% by weight of superabsorbent polymer available as Salsorb CL 15 from Chemdal (United Kingdom). The amount of superabsorbent in the absorbent core may be in the range of from about 5 to about 50 % by weight of the absorbent core. The absorbent core may be compressed or uncompressed, embossed, or calendered.

### Backsheet

The backsheet of the present invention is a body fluid impervious material, typically referred to as a "barrier," which is at least substantially impermeable to liquids. Its exterior forms the garment-facing surface of the absorbent article. The backsheet may be any thin, flexible, body fluid impermeable material, such as, but not limited to, a polymeric film, e.g., polyethylene, polypropylene, or cellophane, or a normally fluid pervious material that has been treated to be impervious, such as impregnated fluid repellent paper or non-woven material, including non-woven fabric material, or a flexible foam, such as polyurethane or cross-linked polyethylene. In the present invention, the backsheet may be a mixture of material and pigment. The thickness of the backsheet may be from about 0.0005 to about 0.002 inch (about 0.013 mm to about 0.051 mm).

Optionally, the backsheet may be breathable, i.e., permits vapor to transpire. Known materials for this purpose include nonwoven materials, monolithic and microporous films in which microporosity is created by, inter alia, stretching an oriented film. Single or multiple layers of permeable films, fabrics, melt-blown materials, and combinations thereof that provide a tortuous path, and/or whose surface characteristics provide a liquid surface repellent to the penetration of liquids may also be used to provide a breathable backsheet.

### Absorbent Structure

An absorbent structure is formed from an absorbent core and a film, which have been laminated together to form a unitary structure. The film may be any water insoluble, biodegradable polymers, such as those used as the cover or alternately the film, may be made from materials, such as, PCL, PLA, and PVOH.

The film may be adhered to the absorbent core by any method known to one of skill in the art. Such methods of adherence include mechanical methods, such as, heating, embossing, crimping, hooks, and the like, and chemical methods, such as, adhesives, including latex, solvents, and the like.

In one embodiment, an absorbent structure can be made of an absorbent core made of a wet laid pulp, available as Valucore® brand from Rayonier, Jessup, GA, which is laminated using hot melt adhesive to a 1 mil layer of PCL (monomer available from Union Carbide/Dow, Midland, MI).

An outer shell (not shown) is water dispersible and may be made of any water soluble, fluid impermeable material. It may be formed from a laminate, composite or blend. It can also be a blend of water soluble and water insoluble materials or a composite of water soluble and water insoluble material. By having the water soluble component in the blend or composite, the water insoluble component will break apart upon exposure to water and agitation.

In one embodiment, a layer of water insoluble, biodegradable material is laminated or coated with a water soluble material. For example, an outer shell can be formed by coating a water-soluble PVOH resin to paper (cellulose fibers) on one side to form a laminated structure. The paper itself is not water soluble but can be biodegraded. PVOH is soluble upon exposure to water.

In another embodiment, a water insoluble material is blended with a water soluble material that acts as a binder. For example, a polyester material may be blended with a water soluble material, such as, PVOH. An example of this embodiment are blends of PCL and PVOH including about 25: about 75, about 50: about 50, and about 75: about 25 weight/weight blends that were melt extruded at about 350° to about 420°F (about 177 to about 216°C) at about 10 RPM into films.

Other examples of water insoluble, biodegradable materials include polyortho esters, polyanhydrides, tyrosine-based polymers, polyphosphoesters, polyetheramides, polyesters including, but not limited to, poly(butylene succinate), poly(butylene succinate-co-adipate), poly(butylene succinate-co-carbonate), poly(butylene succinate-co-terephthalate), poly(ethylene succinate-co-adipate), poly(butylene adipate-co-terephthalate), poly(tetramethylene adipate-co-terephthalate), PCL, PLA, and bacterial polyesters, such as, poly(hydroxyalkanoates). Natural polymers, such as, polysaccharides including starch and their blends, cellulose and their derivatives and biosynthetic polysaccharides may also be used.

Different types of water dispersable papers may also be used. In one embodiment, the outer shell may have two layers: a layer of water soluble paper and a layer of PVOH. In another embodiment, the outer shell may have three layers: a layer of water soluble paper coated on both sides by PVOH. Alternately, the paper pulp may be mixed with a water soluble material. For example, the water soluble paper components may be mixed with PVOH prior to forming the layer of water soluble paper. The PVOH may act as a binder and be used to increase the strength and integrity of the paper layer. For example, Mishima Paper Co., LTD manufactures water soluble paper made from cellulose and CMC that can dissolve in water in about 10 to about 180 seconds. In particular, types A3015 and B3015 are extrusion laminated with PVOH and may be heat sealed to the absorbent structure. Other types of paper may be attached to the absorbent using construction adhesive or attached by other means. In another embodiment, A3015 paper (Mishima Paper Co., LTD) is laminated to the absorbent structure.

### Bonding

The layers of the absorbent article may be, but not necessarily, bonded, e.g., glued or adhered, to an adjacent layer. For example, the underside of the cover layer is adhered to the top side of the absorbent layer. The underside of the absorbent layer is adhered to the top side of the backsheet. As previously stated, any methods known in the art, such as fusion bonding, adhesive attachment, or by any other securement means can be used to bond the individual layers together to form the final absorbent article. Fusion bonding includes heat bonding, ultrasonic bonding, and the like.

Any construction adhesive may be used to attach the layers into a single absorbent article. For example, in one embodiment, the body facing layer is attached to the outer shell with adhesive HL 1491 available from H.B Fuller and Company (St. Paul, MN). The adhesive may be applied by any method known to those skilled in the art.

The absorbent article may be applied to the crotch of a garment by placing the garment-facing surface against the inside surface of the crotch of the garment. Pressure sensitive adhesive may be applied to the garment-facing surface of the absorbent article to help secure it in place. As used herein, the term "pressure-sensitive adhesive" refers to any releasable adhesive or releasable tenacious means. Suitable pressure sensitive adhesives include for example water-based adhesives such as acrylate adhesives. Alternatively, the adhesive may comprise "hot melt" rubber adhesives or two-sided adhesive tape. Preferably, the pressure-sensitive adhesive is adhesive number D-39964-B available from Fuller Co. (Germany). As a non-limiting example, pressure sensitive adhesive strips, swirls, or waves may be applied to help maintain the absorbent article in place. As used herein, the term pressure-sensitive adhesive refers to any releasable adhesive, or releasable tenacious means.

As is customary in the art, a paper release strip, which has been coated on one side, is applied to protect adhesive that may be applied to the garment-facing side of the backsheet. The coating on the paper release strip, which may be silicone, reduces the adherency to the adhesive of the coated side of the release strip. The release strip can be formed from any suitable sheet-like material which, when coated, adheres with sufficient tenacity to the adhesive to remain in place prior to use but which can be readily removed when the absorbent article is to be used.

### Wings

Wings, also called, among other things, flaps, or tabs, may also be part of the absorbent article of the present invention. Wings and their use in sanitary protection articles are described in U.S. Patent. No. 4,687,478 to Van Tilburg; U.S. Patent No. 4,589,876 also to Van Tilburg, U.S. Patent No. 4,900,320 to McCoy, and U.S. Patent No. 4,608,047 to Mattingly. The disclosures of these patents are incorporated herein by reference in their entirety. As disclosed in the above documents, wings are generally speaking flexible and configured to be folded over the edges of the underwear so that the wings are disposed between the edges of the underwear.

The overall dimensions of the absorbent article of the present invention are preferably as follows: length is preferably in the range of about 3 inches (7.63 cm) to about 9 inches (22.9 cm). The maximum width of the anterior portion is preferably in the range of about 1.5 inches (3.81 cm) to 3 inches (7.6 cm).

The absorbent article of the present invention may be used with conventional underwear or may be shaped to conform to thong garments. As used herein, the term thong garment includes, but is not limited to, thong underwear, thong swimming suit bottom, G-strings, Rio cut underwear, Rio-cut swimming suit bottom, Brazilian cut underwear, Brazilian cut swimming suit bottom, and any other garment that exposes the buttocks, having a narrow strip of fabric or a cord that passes between the thighs supported by a waistband, a waist cord, belt or the garment itself.

Other embodiments are also included within the scope of the present invention. For example, inert pigments may be used to produce a colored absorbent article. An example may be light or dark pigment, such as carbon black, blue, green, pink, red, etc. Additionally, patterns including multiple colors and stripes, such as tiger, zebra stripes may be included.

The specification and examples above are presented to aid in the complete and non-limiting understanding of the invention disclosed herein. Since many variations and embodiments of the invention can be made without departing from its spirit and scope, the invention resides in the claims hereinafter appended.

## Claims

1. An absorbent article comprising:
a) a body facing surface and
b) a garment facing surface
wherein at least one embossment is provided on a surface of a material of the absorbent article and the embossment comprises a raised portion having an upper surface that is formed within a depressed area between at least two sidewalls, wherein the surface of the material opposite the embossing pattern is substantially flat.

2. An absorbent article of claim 1, wherein the embossment is provided on a cover, an absorbent core, or both.

3. An absorbent article of claim 2, wherein a coating is deposited on the surface of the material having the embossment.

4. An absorbent article of claim 3, wherein the coating is deposited on a surface selected from the group consisting of an upper surface of the material, the raised portion upper surface, a lateral depressed area floor of the depressed area, a longitudinal depressed area floor of the depressed area, and combinations thereof.

5. An absorbent article of claim 4, wherein the coating is selected from the group consisting of a hot melt adhesive, an ink, a dye, PVOH, hot melt coatings; natural rubber; synthetic rubber; a polyolefin, ethylene vinyl acetate; thermoplastic elastomers, colorants, pigments, fragrances, and mixtures thereof.

6. An absorbent article of claim 1, further comprising an embossing pattern having a plurality of embossments.

7. An absorbent article of claim 6, wherein the embossing pattern is selected from the group consisting of hatch marks, criss-crosses, bars, waves, spirals, swirls, letters, words, numbers, logos, geometric shapes, and combinations thereof.

8. An absorbent article of claim 1, wherein the shape of the raised portion upper surface is selected from the group consisting of flat, curved, pointed, irregular planes, and combinations thereof.

9. An absorbent article of claim 8, wherein the embossment is provided on a cover, an absorbent core, or both.

10. An absorbent article of claim 9, wherein a coating is deposited on the surface of the material having the embossment.

11. An absorbent article of claim 10, wherein the coating is deposited on a surface selected from the group consisting of an upper surface of the material, the raised portion upper surface, a lateral depressed area floor of the depressed area, a longitudinal depressed area floor of the depressed area, and combinations thereof.

12. An absorbent article of claim 11, wherein the coating is selected from the group consisting of a hot melt adhesive, an ink, a dye, PVOH, hot melt coatings; natural rubber; synthetic rubber; a polyolefin, ethylene vinyl acetate; thermoplastic elastomers, colorants, pigments, fragrances, and mixtures thereof.

13. An absorbent article of claim 8, further comprising an embossing pattern having a plurality of embossments.

14. An absorbent article of claim 13, wherein the embossing pattern is selected from the group consisting of hatch marks, criss-crosses, bars, waves, spirals, swirls, letters, words, numbers, logos, geometric shapes, and combinations thereof.

15. An embossment in a material comprising at least one depressed area and a raised portion having an upper surface that is formed within the depressed area between at least two sidewalls, wherein the surface of the material opposite the embossing pattern is substantially flat.

16. An embossment of claim 15, wherein the shape of the raised portion upper surface is selected from the group consisting of flat, curved, pointed, irregular planes, and combinations thereof.

17. A device for embossing comprising a substantially flat female roll and a male roll configured to provide at least one embossment comprising at least one depressed area and a raised portion having an upper surface that is formed within the depressed area between at least two sidewalls, wherein the surface of the material opposite the embossing pattern is substantially flat.

18. A device for embossing of claim 17, wherein the device configured to fit an embossing machine configuration selected from the group consisting of plate to plate, plate to roll and roll to roll.

19. A method of embossing comprising using the device of claim 17 to produce an embossed area.

20. A method of embossing comprising using the device of claim 17 to emboss a material forming a body facing surface of an absorbent article.
